# EUROPEAN PATENT APPLICATION

(11) **EP 2 471 545 A1**
(43) Date of publication of application: **04.07.2012**
(21) Application number: 10774583.8
(22) Date of filing: 04.05.2010
(51) Int. Cl.: A61K 36/63, A01N 65/08, A61K 8/11, B01J 13/16, A61P 33/14, A61P 17/00

(54) **MICROENCAPSULATED COMPOSITION BASED ON SAPONIFIED OLEA EUROPAEA, USE THEREOF AND PRODUCTION METHOD OF SAME**

(30) Priority: 11.05.2009 ES 200930147
(71) Applicant: Mateo Herrero, Maria Pilar, 46007 Valencia (ES)
(72) Inventor: Mateo Herrero, Maria Pilar, 46007 Valencia (ES)
(74) Representative: Ungria Lopez, Javier
(86) International application number: PCT/ES2010/070291
(87) International publication number: WO 2010/130856

(57) **Abstract**

The invention relates to a microencapsulated composition **characterized in that** it comprises 0.01 to 98% by weight of saponified *Olea europaea* and at least one natural repellent selected from a group consisting of tea tree (*Melaleuca alternifolia*) oil, Neem tree oil, Rosemary oil, Bay oil, Eucalyptus oil and Citronella oil or any combination of the aforesaid. Also, the method for obtaining said composition and the use thereof for treating pediculosis will also be an object of protection. One particular embodiment of the invention may additionally comprise a drug for topical treatment of skin disorders.

## Description

### Technical field

The present invention relates to a new composition for treating pediculosis based on saponified *Olea europaea.*

### State of the art prior to the invention

The lice (*Phthiraptera*) are an order of wingless Neoptera insects characterized by being obligate hematophagous ectoparasites of birds and mammals.

This order of insects comprises about 3,000 species and, in humans, they are classified by where they live in: *Pediculus humanus capitis* (head louse), *Pediculus humanus corporis* (body louse) and *Pediculus humanus pubis* (pubic lice).

Specifically, the *Pediculus humanus capitis* (or head louse) is characterized by its ability to reproduce quickly, so that each female is capable of laying from one hundred to three hundred eggs, which stick to the hair of their host. These eggs, which are commonly called nits, are white and take from 4 to 15 days to develop into nymphs. After about 10 days, once converted into adults, the lice are 3 to 4 mm in length and can live for 2 to 3 months.

The skin disorder caused by the infection of lice is called pediculosis. The main symptom of this condition is the itching scalp caused by the irritation of the insect saliva deposited in small bite wound. This irritation, called pruritus, can be so intense that, when scratching, can lead to cause scratches or wounds in the skin.

Commercially, there are different products for treating pediculosis. These products generally consist of lindane or permethrin, although the malathion, benzyl benzoate and other pyrethroids are also effective. Once applied to the scalp, and after about 5 to 10 minutes of action, are able to virtually eliminate all parasites of the same. However, in the case of nits and after a first and a single application, part of the same may remain still habited, requiring repeating the treatment after one week so as to kill the new parasites. Once the treatment is performed, the nits can be removed using a fine comb.

The main problem of lindane is its neurotoxicity, which makes it contraindicated in children under 2 years and pregnant women. In turn, this type of compound, commonly used as pediculicides, has the disadvantage of not effectively destroying the nits or lice. This is because the lice, once they come into contact with water, tend to cling to the hair, closing their airways and preventing an adequate penetration of the insecticide inside. Moreover, the indiscriminate use of pyrethroids is causing the emergence of resistances against thereof, thus reducing its effectiveness for this type of treatments.

Because of these drawbacks, in recent years several investigations have been conducted in order to develop new pharmaceutical products characterized by including no chemical insecticides in their composition. These products include, for example, dimethicone, consisting of a special silicone or natural pyrethrins.

It will therefore be object of the present invention, providing a new composition for the treatment of pediculosis. It will also be an object of the invention the method for obtaining this composition as well as its use to kill, repel or prevent the apparition of lice and/or nits. An additional advantage of this new composition will be the capacity to act against lice and/or nits mechanically and not chemically, thus causing the obstruction of their airways and subsequent death by asphyxiation.

Finally, although this composition is particularly suitable for the treatment of pediculosis, it was found that it could also be used to treat other skin disorders. This will be possible by adding to the composition certain drugs susceptible for being topically used.

In a particularly preferred embodiment, the composition may be used in the treatment of cutaneous leishmaniasis. Leishmaniasis is a zoonotic disease caused by different species of protozoa of the *Leishmania* genus. The clinical manifestations of the disease rang from skin ulcers that heal spontaneously to fatal forms wherein a severe inflammation of the liver and spleen is presented. The *Leishmania* are transmitted by the bite of a female mosquito carrying the *Phlebotomus* genus. The Leishmaniasis treatment is complicated and, generally, can be classified into first- and second-line treatments. The first-line treatment is based on pentavalent antimony drugs (SbV), such as sodium stibogluconate (Pentostam). However, due to the increasingly appearance of resistances to these types of drugs, the second-line treatments have been developed in recent years, which are based on, for example, the use of pentamidine isiotonate or amphotericin B (in particular liposome formulation). The Amphotericin B is the chosen treatment of visceral leishmaniasis in regions that are highly resistant to treatment with sodium stibogluconate, and is carried out both topically and visceral (ES 2212904). Therefore, an additional object of this invention will be the use of the composition based on *Saponified olea europaea* for the topical treatment of cutaneous leishmaniasis, for which the composition will additionally comprise Amphotericin B.

Similarly, it will be possible to use the composition for the topical treatment of various skin disorders, by adding specific drugs for the treatment of such diseases.

### Description of the invention

The present invention relates to a new composition characterized by comprising from 0.01 to 98% by weight of *Saponified olea europaea* and at least one natural repellent selected from the group consisting of Tea tree oil (*Melaleuca alternifolia*) oil, Neem oil, Rosemary, Laurel oil, Eucalyptus oil and Citronella oil or any combination of the aforesaid.

The percentage of said natural repellent is preferably comprised between 0.01 and 5% by weight of the total composition and its presence allows carrying out a preventive control for the appearance of lice and/or nits in the hair.

Regarding the percentage of *Saponified olea europaea,* preferably, said percentage will be comprised between 30 and 70%, more preferably from 40 to 60% by weight of the total composition.

In turn, in particular, the composition may also comprise water, preferably in a percentage comprised between 0.01 and 0.98%, more preferably between 10 and 50%, and most preferably between 20 and 40% by weight of the composition.

Similarly, in particular, the composition may additionally comprise at least one preservative, which is preferably selected from a group consisting of BHT (butylhydroxytoluene), BHA (3-tert-butyl-4-hydroxyanisole), EDTA (ethylene diamine tetra-acetic acid), α-tocopherol, ethoxyquin and at least one derivative of a fatty acid, or any combination of the aforesaid. Among the fatty acid derivatives, esters of long chain fatty acids, mono and diglycerides of fatty acids or glycerol esters of fatty monoacids are preferably used. In turn, preferably, the percentage of such preservative is comprised between 0.01 and 98%, more preferably between 0.05 and 30%, and most preferably between 0.1 and 5% by weight of the composition.

Similarly, in a further embodiment of the invention, the composition may further comprise other type of compounds such as, for example, glycerin, preferably in a percentage comprised between 0.01 and 98%, preferably between 10 and 40%, and more preferably between 20 and 30% by weight of the composition, as well as at least one salt, which will be preferably selected from a group consisting of potassium chloride, sodium hydroxide, potassium hydroxide or any combination of the aforesaid. The percentage of such salt will be comprised between 0.01 and 98%, preferably between 0.1 and 25% and more preferably between 0.5 and 10% by weight of the composition.

One of the advantages of this new composition obeys that it is totally natural and biodegradable; therefore it does not entail any problems of sensitization caused by inhalation or contact. Also, although particularly, the composition may additionally comprise a certain percentage of alcohol, such percentage will be preferably limited to a percentage comprised between 0.01 and 5% by weight of the total composition. As a result, additional problems such as irritation of the scalp, wounds or mucous membranes will be prevented, as well as potential hazards of ignition or explosion of products comprising said composition.

It will, in turn, object of this invention, the production method of this new composition based on *Saponified olea europaea.*

This method involves, in the first place, the saponification of *Olea europaea.* The *Olea europaea,* according to INCI (International Nomenclature of Cosmetic Ingredients) corresponds to olive oil, extracts and its physically modified derivatives. In order to obtain the composition object of the invention, the *Olea europaea* is saponified by using a basic compound, preferably potassium hydroxide, at a percentage that depends on the type of *Olea europaea* used, and which is a function of the saponification index. Such saponification index must be preferably comprised between 185 and 195 mg KOH/g *Olea europaea.* Then, once the *Olea europaea* has been saponified, the remaining ingredients of the composition are added, as described above.

Also, a particular embodiment of the invention may further comprise an additional step of microencapsulating the composition, which can be done using one of the following methods:
i) Firstly, by interfacial polymerization. This method consists in the polymerization of at least one monomer at the interface of two immiscible substances, resulting in a membrane that constitutes the wall of the microcapsule. In turn, such interfacial polymerization may include the following substeps:
   a) dispersing an aqueous solution of at least one watersoluble reactant into an organic phase, preferably oil, resulting in an emulsion;
   b) forming a polymeric membrane on the surface of the water droplets of the emulsion of step (a) resulting in microcapsules;
   c) separating the microcapsules from the organic phase by washing with water, resulting in an aqueous suspension.
   With respect to the reactant used in step (a), this will be preferably selected from a group consisting of alkali metals of Group IA and derivatives thereof, chlorates, perchlorates, sulfates, sodium hydroxide and acrylic styrene dispersions or any combination of the aforesaid. Among the sulfates the calcium sulfate will be preferably used. Regarding the required amount of reactant, this will be preferably comprised between 0.1 and 2% by weight of the composition.
ii) By polymer incompatibility. In turn, this method for forming microcapsules is based on the use of the phenomenon of phase separation in a mixture of two chemically different and incompatible polymers in the same solvent. In this way, the material to be encapsulated only interacts with one of the two polymers, which is absorbed on the surface of the material to be encapsulated forming a film that encompasses it.

In this case, the polymer used is the saponified oil itself, which is added in at least one solvent, preferably selected from water and alcohol, preferably, ethyl or isopropyl alcohol. The added amounts of the same will be those required to obtain a final size of microcapsules preferably comprised between 1 and 10 micrometers.

The fact of using the saponified *Olea europaea* in the composition is an additional advantage of the invention by allowing its application as liquid soap, which avoids having to use this type of product after its performance in the hair. By being a liquid soap, its application will be conducted as any other shampoo or liquid soap, applying it to the scalp and letting it act for 15 to 90 minutes, preferably from 30 to 60 minutes. Preferably, an additional coverage will be used on the hair, like a skullcap, during its application, after which the composition will be removed by applying water. After rinsing the hair, a comb specially designed for the removal of nits or lice will be passed. For an effective treatment of the composition, this process should be repeated after 8 to 15 days, preferably 10 to 12 days, in order to permanently remove lice coming from the nits that might have survived the first application of the composition.

It will, therefore, be a further object of the present invention, the use of the composition to kill, repel or prevent the appearance of lice and/or nits in the hair.

Finally, as mentioned above, the composition object of the invention may include other additional drugs, so that it may be used to treat different kinds of skin disorders. Preferably, the composition may additionally include Amphotericin B, in this case being suitable for the topical treatment of cutaneous leishmaniasis.

Similarly, other type of drugs that can be topical used to treat skin disorders may include, but are not limited to:
a) Topical antihistamines, which will be preferably selected from a group consisting of diphenhydramine, dimethindene, promethazine and tripelennamine, and will be effective for the treatment of allergic and contact dermatitis;
b) topical corticosteroids preferably selected from a group consisting of hydrocortisone acetate, clobetasone, butyrate, fluocortin, hydrocortisone butyrate, hydrocortisone propionate, betamethasone, budesonide, desoximetasone, clobetasol and halcinonide, which will be used for the treatment of allergic and contact dermatitis. These diseases include, for example, seborrheic dermatitis, erythema with minimal scaling or lichenification, nummular dermatitis, mild psoriasis, discoid lupus erythematosus, plaque psoriasis, simplex chronicus lichen, planus hypertrophic lichen or chronic dermatitis of the hands, among other;
c) Antimycotic or antifungal agents for the treatment of dermatophytosis or dermatophyte infections, which will be selected from a group consisting of ciclopirox olamine, amorolfine, allylamines, tolnaftate, salicylic acid and imidazole derivatives. The latter will be preferably selected between bifonazole, clotrimazole, econazole, flutrimazole, ketoconazole, miconazole, oxiconazole, tioconazole and sertaconazole;
d) Antimycotic or antifungal agents for the treatment of Candida, which will be selected from ciclopirox olamine, amorolfine, amphotericin B, nystatin, methylrosaniline and imidazole derivatives, preferably ketoconazole;
e) 28% Tioconazole or amorolfine for the treatment of onychomycosis;
f) Imidazoles or tolnaftate for the treatment of tinea versicolor;
g) Cicatrizers selected from beclapermine or gotu kola;
h) Proteolytic enzymes selected from a group consisting of clostridiopeptidase A, chymotrypsin and trypsin.

Following, a set of examples are collected in a not limitative manner, corresponding to particular embodiments of the present invention.

### Example 1.- Pediculicide composition

We proceeded to make the composition object of the invention. To do this, after including the primary ingredients (*Olea europaea* and potassium hydroxide) in process vessels, the saponification step of the *Olea europaea* was carried out, thus obtaining a first phase of the composition. Once this first phase was obtained, we proceeded to include into the same vessels used in the previous step the other ingredients of the composition, according to the following percentages:

**Table 1. Pediculicide composition**

| | |
|---|---|
| Water | 30% |
| Saponified *Olea europaea* | 42.8% |
| Glycerin | 23.5% |
| Ethyl alcohol | 1.5% |
| Potassium chloride | 1.5% |
| Tea tree oil | 0.5% |
| BHT | 0.2% |

### Example 2.- Characterization of the composition

We proceeded to characterize the composition obtained from the production method described in Example 1, according to 93/112/EC and ISO 11014-1, thus obtaining the following data and specifications:

**Table 2. Specification and other data of the composition**

| Appearance: | Transparent/translucent liquid | Anhydrous Soap: | 33.0 +/- 2% |
|---|---|---|---|
| Color and Odor: | Characteristic | Humidity: | 37.0 +/- 3% |
| Active matter: | 33.0 +/- 2% | Free alkali: | 0.04% max. (NaOH) |
| pH: | 9.8-10.2 (5% in water) | Fat content: | 31.0 +/- 2% |
| Density: | 1010 kg/m³ | Free acidity: | 1.3% max. (Oleic acid) |
| Cloud Point: | 15 ° +/- 1 | Vapor Pressure: | < 0.1 hPa (20°C) |
| Potassium chloride: | 1.5 +/- 0.5% | | |

### Example 3.- Dermal irritation tests

On the other hand, dermal irritation tests were conducted "in vitro" using the analytical method OECD *Guideline 431* of March 27, 2002, and the cellular model *Epiderm Skin Model (EPI-200),* using as DMEM and MatTek culture medium for an hour of exposure of the sample at 100% of its concentration. According to the obtained results, the percentage of viability after one hour is 85.77%, therefore it is concluded that the composition is not irritating to the skin. It is considered that a product is irritating if the viability after one hour of exposure is less than 15%, the percentage of viability being defined as the ratio between the absorption of the sample and the absorption of negative control (corresponding to sterile water and non-pyrogenic) multiplied by one hundred.

### Example 4.- Tests of efficiency

Finally, we proceeded to an analysis of the effectiveness of the composition against *Pediculus humanus capitis,* applying it by immersion according to an internal methodology for obtaining the Knock down after 10 minutes and mortality at 18 hours, comparing it with a negative control. The results obtained were as follows:

**Table 3. Pediculicide effectiveness**

| Sample: | Immersion time (min) | % Tumbling after 10 min (mean +/- σ) | % Mortality after 18 hours (mean +/- σ) |
|---|---|---|---|
| Composition (1): | 2 | 10 +/- 10 | 50 +/- 2 |
| Composition (2): | 10 | 36.7 +/- 12.5 | 76.7 +/- 6.9 |
| Composition (3): | 30 | 70 +/- 14 | 90 +/- 8.2 |
| Water (negative control): | 2 | 0 | 0 |
| Water (negative control): | 10 | 0 | 15.0 +/- 2.0 |
| Water (negative control): | 30 | 3.3 +/- 2.7 | 17.5 +/- 2.5 |

As shown by these results, the composition object of the invention has a high efficiency tumbling and mortality, specially, after being exposed for 30 minutes.

### Example 5.- Composition for the treatment of leishmaniasis

We proceeded to prepare a composition according to the method described in Example 1. In this case, the percentages used for the various components were as follows:

**Table 4. Composition for the treatment of leishmaniasis**

| | |
|---|---|
| Water | 30% |
| Saponified Olea Europaea | 44.5% |
| Glycerin | 22.3% |
| Ethyl alcohol | 1.5% |
| Potassium chloride | 1.0% |
| Amphotericin B | 1.0% |
| BHT | 0.2% |

This composition was effective for the treatment of leishmaniasis.

### Example 6.- Composition for the treatment of dermatophytosis

Finally, we proceeded to prepare a composition according to the method described in Example 1, according to the following percentages:

**Table 5. Composition for the treatment of dermatophytosis**

| | |
|---|---|
| Water | 30% |
| Saponified Olea Europaea | 43.5% |
| Glycerin | 22.3% |
| Ethyl alcohol | 1.0% |
| Potassium chloride | 1.0% |
| Amorolfine | 2.0% |
| BHT | 0.2% |

This composition was effective for the treatment of dermatophytosis.

## Claims

1. Composition **characterized by** comprising from 0.01 to 98% by weight of saponified *Olea europaea* and at least one natural repellent selected from a group consisting of tea tree oil, Neem tree oil, Rosemary oil, Bay oil, Eucalyptus oil and Citronella oil or any combination of the aforesaid.

2. Composition according to claim 1, **characterized in that** the repellent is comprised between 0.01 and 5% by weight of the composition.

3. Composition according to one of claims 1 or 2, **characterized in that** it also comprises at least one alcohol.

4. Composition according to one of claims 1, 2 or 3, **characterized in that** it further comprises at least one preservative.

5. Composition according to claim 4, **characterized in that** said preservative is selected from a group consisting of butylhydroxytoluene, 3-tert-butyl-4-hydroxyanisole, ethylene-diamine-tetra-acetic acid, α-tocopherol, ethoxyquin and at least one derivative of a fatty acid, or any combination of the aforesaid.

6. Composition according to any one of the preceding claims, **characterized in that** it further comprises glycerin.

7. Composition according to any one of the preceding claims, **characterized in that** it further comprises at least one salt selected from a group consisting of potassium chloride, sodium hydroxide, potassium hydroxide or any combination thereof.

8. Composition according to any one of the preceding claims, **characterized in that** it further comprises Amphotericin B.

9. Composition according to any one of claims 1 to 7, **characterized in that** it further comprises at least one drug selected from a group consisting of topical antihistamines, topical corticosteroids, antimycotic agents, cicatrizers and proteolytic enzymes, or any combination of the aforesaid.

10. Production method of the composition according to any one of the preceding claims, **characterized in that** it comprises:
a) the saponification of *Olea europaea* by using a basic compound, resulting in a first phase of the composition;
b) the mixture of said first phase of the composition with the other ingredients of the composition.

11. Method according to claim 10, **characterized in that** it further comprises a step for microencapsulating the composition.

12. Method according to claim 11, **characterized in that** the microencapsulation is carried out by interfacial polymerization.

13. Method according to claim 11, **characterized in that** the microencapsulation is carried out by polymer incompatibility.

14. Microcapsule **characterized by** comprising a composition based on saponified *Olea europaea* according to any one of claims 1 to 9.

15. Composition according to any one of claims 1 to 7, for use in the treatment of pediculosis.

16. Composition according to claim 8, for use in the topical treatment of cutaneous leishmaniasis.

17. Composition according to claim 9, for use in the treatment of skin disorders.
